# EUROPÄISCHE PATENTSCHRIFT

(11) **EP 3 241 575 B1**
(45) Veröffentlichungstag und Bekanntmachung des Hinweises auf die Patenterteilung: **02.06.2021**
(21) Anmeldenummer: 17168047.3
(22) Anmeldetag: 25.04.2017
(51) Int. Cl.: A61M 1/14, A01N 25/26, C09D 5/14, A61L 2/232

(54) **MEDIZINTECHNISCHES GERÄT MIT ANTIMIKROBIELLER OBERFLÄCHENBESCHICHTUNG SOWIE VERFAHREN ZUR BEKÄMPFUNG VON MIKROORGANISMEN AUF DER OBERFLÄCHE EINES SOLCHEN GERÄTS**
MEDICAL DEVICE WITH ANTIMICROBIAL SURFACE COATING AND METHOD FOR COMBATING MICRO-ORGANISMS ON THE SURFACE OF SUCH A DEVICE
APPAREIL MÉDICAL COMPRENANT UN REVÊTEMENT DE SURFACE ANTIMICROBIEN ET PROCÉDÉ DE LUTTE CONTRE LES MICROORGANISMES SUR LA SURFACE D'UN TEL APPAREIL

(30) Priorität: 03.05.2016 DE 102016108198
(43) Veröffentlichungstag der Anmeldung: 08.11.2017
(73) Patentinhaber: B. Braun Avitum AG, 34212 Melsungen (DE)
(72) Erfinder: SCHÄFER, Oliver, 36286 Neuenstein (DE); SCHLEICHER, Christian, 36160 Dipperz (DE)
(74) Vertreter: Winter, Brandl - Partnerschaft mbB

(56) Entgegenhaltungen:
- EP-A1- 2 574 353
- WO-A1-2011/130124
- CN-A- 105 368 274
- CN-A- 105 419 559
- DE-A1-102005 042 181
- DE-A1-102014 215 353
- DE-U1-202005 014 409
- US-A1- 2008 051 493
- US-A1- 2008 148 994
- US-A1- 2010 297 206
- US-A1- 2011 171 062

## Beschreibung

Die Erfindung betrifft ein medizintechnisches Gerät zur extrakorporalen Blutbehandlung, mit einer antimikrobiellen Oberflächenbeschichtung, sowie ein Verfahren zur Bekämpfung von Mikroorganismen auf einer Oberfläche eines medizintechnischen Geräts nach der Erfindung.

Wachstum und Verbreitung von Mikroorganismen auf Oberflächen medizintechnischer Geräte stellen unerwünschte Effekte dar, die in einer jeweiligen Behandlungsumgebung, wie Krankenhäuser, Labore, Arztpraxen und dergleichen, ein erhebliches Hygienerisiko darstellen und den Gebrauchswert von solchen medizintechnischen Geräten und Gegenständen beeinträchtigen. Üblicherweise werden Mikroorganismen in solchen Umgebungen durch entsprechende Reinigung und Desinfektion bekämpft.

Darüber hinaus ist aber erwünscht, dass sich Mikroorganismen erst gar nicht oder nur stark verzögert verbreiten und festsetzen. Dieses Ziel wird auch in der WO 2011/130124 A1 verfolgt, die ein Verfahren zum Immobilisieren von Farbstoffen und antimikrobiellen Mitteln auf Kunststoffoberflächen von medizintechnischen Geräten offenbart. Dabei wird ein Kunststoffgehäuse mit einer Mehrzahl funktioneller Gruppen behandelt, an diesen eine Verbindungsgruppe angelagert und daran wiederum ein antimikrobielles Agens. Es kann ein nachteiliger Effekt entstehen, der darin besteht, dass bestimmte Eigenschaften des Kunststoffs, wie zum Beispiel Alterungsbeständigkeit, Zugfestigkeit, Schlagfestigkeit etc., durch die Einlagerung von Additiven in die Kunststoffmatrix negativ beeinflusst werden.

Die DE 10 2005 042 181 A1 offenbart in diesem Zusammenhang einen Formkörper, insbesondere für Geräteteile, Geräteoberschalen, Geräteinnenteile, Gerätezubehörteile, Gerätekomponenten, Abformmaterialien, Füllungsmaterialien für medizintechnische Geräte und/oder medizinische Produkte, der wenigstens abschnittsweise Keimansiedlungen abweisend ausgebildet ist. Ferner wird offenbart ein Medizingerät, das wenigstens einen solchen Formkörper aufweist, wie auch ein Verfahren zur Herstellung eines solchen Formkörpers. Ein Ansatz dabei ist, die Oberfläche durch Verringerung der Adhäsion keimabweisend auszubilden. Ein weiterer Ansatz ist, Biozide in den Kunststoff einzubauen.

Weiterer Stand der Technik ist aus der EP 2 574 353 A1 bekannt, die einen Impeller eines Gefäßanschlusssystems für eine Dialysebehandlung offenbart.

Ausgehend von dem vorstehend beschriebenen Stand der Technik liegt der vorliegenden Erfindung die Aufgabe zugrunde, die zuvor angeführten Nachteile zu beseitigen, insbesondere ein medizintechnisches Gerät mit antimikrobiellen Eigenschaften zur Verfügung zu stellen, das einfach herzustellen ist. Mechanische Eigenschaften des Gehäusematerials sollen durch die antimikrobiellen Eigenschaften nicht negativ beeinflusst werden.

Nach der Erfindung wird diese Aufgabe gemäß den Merkmalen der unabhängigen Ansprüche gelöst. So ist ein medizintechnisches Gerät der eingangs genannten Art dadurch gekennzeichnet, dass die antimikrobielle Oberflächenbeschichtung eine Pulverbeschichtung ist, die Additive mit antimikrobieller Wirkung aufweist, wobei die Additive metallorganische Stoffe sind, wobei die Additive ionisierende und photokatalytische Wirkung haben, sodass durch die Additive Ionen und Radikale gebildet werden, die jeweils von der Pulverschicht emittiert werden. Verfahrensseitig wird die Aufgabe gelöst durch ein Verfahren zur Bekämpfung von Mikroben und Mikroorganismen auf einer Oberfläche eines medizintechnischen Geräts nach der vorliegenden Erfindung, wobei die Additive der Pulverbeschichtung des medizinischen Geräts Ionen und Radikale ausbilden und emittieren, die Stoffwechselsysteme von Mikroben und/oder Mikroorganismen angreifen. Eine solche antimikrobielle Pulverbeschichtung verhindert besonders einfach und wirksam die Entstehung und Entwicklung von Mikroorganismen, insbesondere von Bakterien und Pilzkulturen.

Nach der Erfindung kann die Pulverbeschichtung auf einem Gerätegehäuse eines Medizingerätes, dessen bestimmungsgemäßer Einsatz sich in der extrakorporalen Blutbehandlung findet, aufgebracht sein. Unter dem Begriff Mikroorganismen können im Rahmen der Erfindung auch Viren verstanden werden. Die Wirkungsweise der antimikrobiellen Oberfläche beruht auf in der Beschichtung eingebaute Bestandteile (Additive), welche gegenüber Mikroorganismen hoch wirksam, für den menschlichen Organismus aber unbedenklich sind.

Da es sich um eine Beschichtung in Form einer Pulverbeschichtung handelt, welche auf das Grundmaterial aufgebracht wird, werden hierdurch keine Eigenschaften des Gehäuses verändert, welche für dessen Funktionalität relevant sind. Zu nennen sind in diesem Zusammenhang zum Beispiel mechanische Eigenschaften des Gehäusematerials oder EMV-Eigenschaften (elektromagnetische Verträglichkeit) der gehäusebildenden Bauteile. Dies ist eine weitere Unterscheidung zu antibakteriellen Oberflächen, welche zum Beispiel durch integrierte Nanopartikel in Kunststoffen erzeugt werden. Bei diesem Prinzip werden auch die Eigenschaften des gehäusebildenden Grundmaterials beeinträchtigt.

Vorteilhafte Ausführungsformen der Erfindung sind in den Unteransprüchen beansprucht und werden nachfolgend näher erläutert.

Die Pulverbeschichtung kann nur in oder auf Teilbereichen oder vollflächig auf das Gerät aufgebracht sein. Zum Beispiel kann sie nur an solchen Bereichen vorhanden sein, mit denen Patienten oder Bedienpersonal in Berührung kommen, wie Außenbereiche oder Bedieneinheiten. Es liegt jedoch auch im Rahmen der Erfindung, wenn die Pulverbeschichtung vollflächig aufgebracht und ausgebildet ist.

Eine Ausführungsform der Erfindung ist dadurch gekennzeichnet, dass die Pulverbeschichtung auf eine metallische Oberfläche aufgebracht ist. Alternativ oder zusätzlich kann das medizintechnische Gerät ein Gerätegehäuse aufweisen und die Pulverbeschichtung zumindest teilweise auf dem Gerätegehäuse aufgebracht sein. Es ist ein besonderer Vorteil, dass die Erfindung mit metallischen Gehäusen oder Gehäuseteilen verwendet werden kann, da bei solchen eine Einlagerung von mikrobiologisch aktiven Substanzen in die Werkstoffmatrix, wie dies bei Kunststoffen praktiziert wird, nicht möglich oder nur unter Schwächung des Grundstoffmaterials möglich ist.

Nach der Erfindung weist die Pulverbeschichtung Additive auf, die lonen- und Radikale-Emittenten sind. Auf diese Weise können kontinuierlich, vorzugsweise permanent, insbesondere fortwährend, neue Ionen und Radikale gebildet und von der Pulverbeschichtung emittiert werden. Die emittierten Ionen und Radikale greifen die Stoffwechselsysteme der Mikroorganismen oder Zellen an, so dass diese absterben. Die antimikrobielle Pulverbeschichtung verhindert so deren Ausbreitung. Durch eine permanente Bildung neuer Ionen und/oder Radikale ist im Gegensatz zu anderen Prinzipien, wie z.B. dem Einsatz von reinem/ungebundenem Nanosilber, ein deutlicher Unterschied hinsichtlich des Langzeiteffekts messbar.

Die Bestandteile oder Additive, die die Pulverbeschichtung aufweist, sind erfindungsgemäß metallorganische Stoffe mit ionisierender und photokatalytischer Wirkung.

In diesem Zusammenhang sind Additive, auch Hilfsstoffe oder Zusatzstoffe genannt, Stoffe, die Produkten in geringen Mengen zugesetzt werden, um bestimmte Eigenschaften zu erreichen oder zu verbessern. Hier haben die Additive die Aufgabe, die antimikrobiell wirkenden Stoffe dauerhaft in der Struktur des Trägermaterials zu verankern, um einen Langzeiteffekt zu erzielen. Antimikrobiell wirkende Stoffe sind z.B. Metallkolloide aus Silber, Metallkolloide aus Kupfer, Titandioxid, Zinkoxid als biozides Additiv etc., oder auch antimikrobielle Oligomere.

Die Pulverbeschichtung kann insbesondere Trägerstoffe umfassen, an denen Ionenemittenten und/oder Katalysatoren anhaften. Anhaften in diesem Sinne ist unter anderem als verbunden, chemisch verbunden, etc. zu verstehen.

Es ist ein besonderer Vorteil, dass mittels der Pulverbeschichtung eine Oberflächengestaltung zum Beispiel durch eine besondere Struktur oder Farbgestaltung bewirkt werden kann, während gleichzeitig die genannte antimikrobielle Wirkung appliziert wird. Des Weiteren können durch die Pulverbeschichtung neben der angestrebten antimikrobiellen Wirkung weitere Wirkungen erzielt werden, wie zum Beispiel Korrosionsschutz, etc.

Die Erfindung wird im Folgenden anhand einer beispielhaften, nicht einschränkenden und in der angehängten Figur gezeigten Ausführungsform näher erläutert. Dabei zeigt
Figur 1 eine schematische Darstellung eines Ausschnitts einer Vorrichtung zur extrakorporalen Blutbehandlung als Beispiel für ein medizintechnisches Gerät nach der Erfindung;
Figur 2 eine Darstellung der Wirksamkeit aufgetragen über der Zeit von Nano-Silber;
Figur 3 eine Darstellung der Wirksamkeit aufgetragen über der Zeit von antimikrobiellen Additiven nach der Erfindung;
Figur 4 eine Darstellung eines photokatalytischen Prozesses; und
Figuren 5A und 5B eine Darstellung der Einbindung von reinen/ungebundenen Nanopartikeln in eine Gitternetzstruktur eines Trägermaterials bzw. eine Darstellung der Einbindung von Additiven, welche die antimikrobiellen Wirkstoffe in eine Gitternetzstruktur eines Trägermaterials einbinden.

Gezeigt ist im Wesentlichen der gesamte extrakorporale Blutkreislauf der Vorrichtung. Dieser weist eine arterielle Blutleitung 1 auf, mittels der Blut von einem nicht gezeigten Patienten zu einer Peristaltikpumpe 2 der Behandlungsvorrichtung geführt wird. Vor der Peristaltikpumpe 2 ist ein arterieller Druckabnehmer 3 vorgesehen, mit dem der Druck vor der Peristaltikpumpe 2, also der niederdruckseitige Druck gemessen wird. Hochdruckseitig der Peristaltikpumpe 2 führt eine Hochdruckblutleitung 4 zu einem arteriellen Blutfänger 5. Unmittelbar am Ausgang der Peristaltikpumpe 2 kann mittels einer Zuleitung 6 und einer Pumpe 7 Zusatzstoff in das im System befindlichen Blut gegeben werden, z.B. Heparin zur Blutverdünnung.

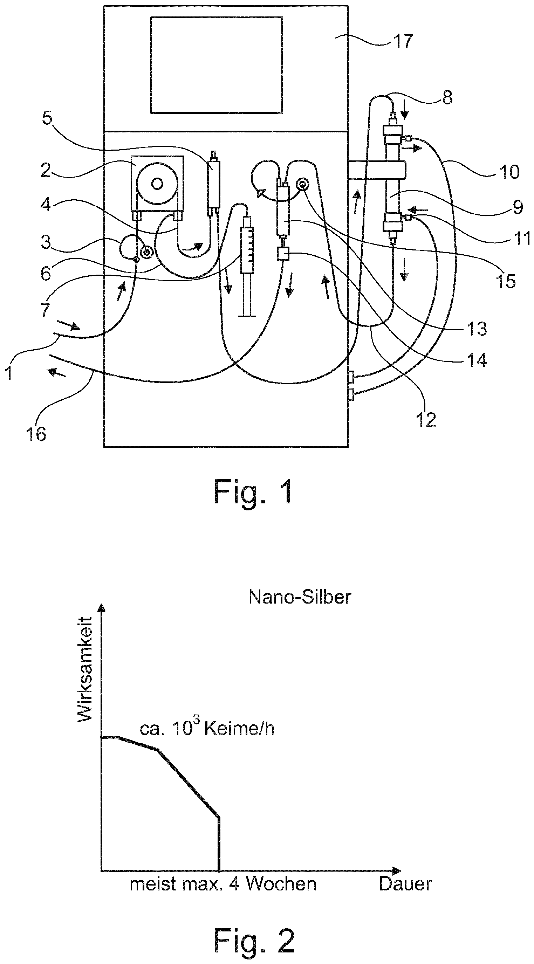

Vom arteriellen Blutfänger 5 führt eine Leitung 8 unter Hochdruck stehendes aber noch unbehandeltes, mit Schlackenstoffen belastetes Blut zu einem Dialysator 9. Diesem wird eingangsseitig Dialysat über eine Dialysatzuleitung 10 zugeführt. Im Dialysator 9 wird Blut in bekannter Weise mittels des Dialysats behandelt, z.B. gereinigt. Verbrauchtes Dialysat wird über eine Dialysatableitung 11 vom Dialysator 9 entfernt und einer nicht dargestellten Entsorgung oder Aufbereitung zugeführt. Behandeltes Blut wird mittels einer Blutableitung 12 vom Dialysator 9 zu einem venösen Luftfänger 13 geleitet, wo mittels einer Luftfalle 14 Luft abgeschieden wird. Am venösen Luftfänger 13 ist ein venöser Druckaufnehmer 15 vorgesehen, mit dem der venöse Druck, also der hochdruckseitige Druck, erfasst wird. Von der Luftfalle 14 wird behandeltes Blut über eine venöse Blutleitung 16 zurück zum Patienten geleitet. Dargestellt in Figur 1 ist auch eine Einheit 17 zur Überwachung und Steuerung der Vorrichtung. Die Vorrichtung zur extrakorporalen Blutbehandlung ist mit einem Gehäuse gekapselt, das zumindest teilweise als Blechformteil ausgebildet ist.

Nach der Erfindung ist zumindest das Gehäuse mit einer Pulverbeschichtung versehen. Diese kann nur in Teilbereichen oder vollflächig aufgebracht sein. Die Pulverbeschichtung kann aber zusätzlich auf weiteren Einheiten der Vorrichtung vorgesehen und aufgebracht sein, wie zum Beispiel der Steuerungseinheit 17.

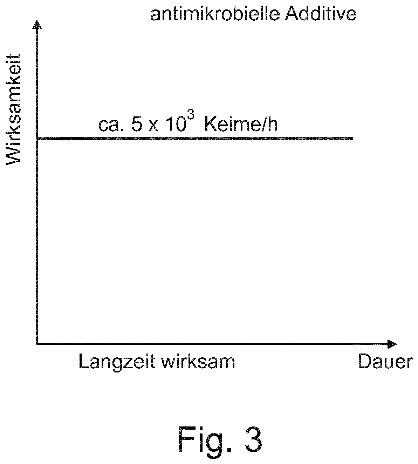

Figur 2 zeigt in einem Diagramm die antimikrobielle Wirksamkeit von Nano-Silber. Figur 3 zeigt in einem ähnlichen Diagramm die antimikrobielle Wirksamkeit nach der Erfindung. Dabei sind die Zeitdauer jeweils auf der Abszisse und die Wirksamkeit jeweils auf der Ordinate aufgetragen. Zu erkennen ist, dass die maximale Wirksamkeit von Nano-Silber bei etwa 10³ Keimen pro Stunde liegt, die nach der Erfindung bei etwa 5 mal 10³ Keimen pro Stunde. Die Wirksamkeit von Nano-Silber fällt nach kurzer Zeit zunächst langsam, dann immer schneller ab und erliegt nach ca. vier Wochen. Die Wirksamkeit nach der Erfindung hingegen hält über einen langen Zeitraum an und nimmt nicht ab.

Die antimikrobielle Wirkung des Nanosilbers beruht auf der Bildung von Silberionen (Ag+) an der Oberfläche von Silbernanopartikeln bzw. Silberkolloiden. Durch das besondere Verhältnis der Silberkolloide von Größe und Oberfläche lässt sich ein ausgeprägter Langzeiteffekt verwirklichen. Die gebildeten Silberionen wirken in verschiedener Weise schädigend auf Einzeller, wie Bakterien, Hefen, Pilze und Viren. Die starke antimikrobielle Wirksamkeit von Nanosilber wird mit dessen Fähigkeit in Verbindung gebracht, Zellwände und Zellmembranen durchdringen zu können und im Zellinnern zu wirken. In vitro wirkt kolloidales Silber auch gegen Viren, indem sich Nanosilberpartikel an deren Oberfläche binden und die Bindung der Viren an Wirtszellen unterdrücken. Weitere antimikrobielle Metallkolloide sind zum Beispiel Kupferkolloide.

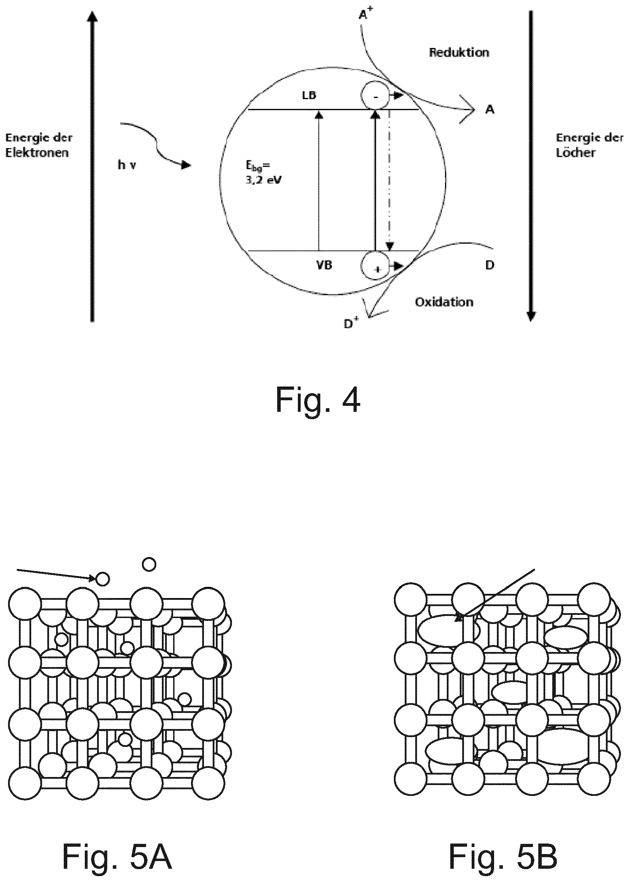

Die antimikrobielle Wirkung von z.B. Nanopartikel aus Titandioxid beruht auf einem photokatalytischen Prozess, welcher eine dauerhafte Reaktion in Verbindung mit Lichteinstrahlung bewirkt. Dabei wirkt das Titandioxid als Photokatalysator durch dauerhafte photochemische Anregung, z.B. durch Tageslicht. Dadurch finden physikalische und chemische Reaktionen (Reduktionen, Oxidationen) statt (siehe Fig. 4). Im Falle von Titandioxid entstehen hierbei Hydroxyl-Radikale, Superoxidanionen-Radikale und Wasserstoffperoxid. Die Radikale sind in der Lage, mit organischen Stoffen zu reagieren und diese zu oxidieren. Es wird angenommen, dass eine antimikrobielle Wirkung auf Mikroorganismen von den Hydroxyl-Radikalen ausgeht.

Die antimikrobielle Wirkung von antimikrobiellen Oligomeren beruht auf einer direkten mikrobiziden Wirkung der antimikrobiellen Oligomeren, welche langsam und in geringen Mengen aus der Polymermatrix des Trägermaterials austreten können und somit diese mikrobizide Wirkung in Form einer Depot- oder Retardverbindung freisetzt. Bevorzugt werden die antimikrobiellen Oligomere hergestellt aus einem oder mehreren Monomeren ausgewählt aus der Gruppe Methacrylsäure-2-tert.-butylaminoethylester, Methacrylsäure-2-diethylaminoethylester, Methacrylsäure-2-di-ethylaminomethylester, Acrylsäure-2-tert.-butylaminoethylester, Acrylsäure-3-dimethylaminopropylester, Acrylsäure-2-diethylaminoethylester, Acrylsäure-2-dimethylaminoethylester, Dimethylaminopropylmethacrylamid, Diethylamino-propylmethacrylamid, Acrylsäure-3-dimethylaminopropylamid, 2-Methacryloyloxyethyltrimethylammoniummethosulfat, Methacrylsäure-2-diethylaminoethylester, 2-Methacryloyloxyethyltrimethylammoniumchlorid, 3-Methacryloylaminopropyltrimethylammonium-chlorid, 2-Methacryloyloxyethyltrimethylammoniumchlorid, 2-Acryloyloxyethyl-4-benzoyldimethylammoniumbromid, 2-Methacryloyloxy-ethyl-4-benzoyldimethylammoniumbromid, Allyltriphenylphosphoniurnbromid, Allyltriphenylphosphoniumchlorid, 2-Acrylamido-2-methyl-1-propansulfonsäure, 2-Diethylaminoethyl-vinylether und/oder 3-Aminopropylvinylether.

In den Figuren 5A und 5B ist die Einbindung von reinen/ungebundenen Nanopartikeln in eine Gitternetzstruktur eines Trägermaterials und die Einbindung von Additiven, welche die antimikrobiellen Wirkstoffe in eine Gitternetzstruktur eines Trägermaterials einbinden, dargestellt.

Der Effekt besteht hauptsächlich darin, dass eingelagerte Nanopartikel aufgrund ihrer Größe aus der Gitternetzstruktur des Trägermaterials entweichen können, während die antimikrobiellen Additive die Wirkstoffe direkt in die Gitternetzstruktur einarbeitet und durch die Größe in der Gitternetzstruktur des Trägermaterials verankert. (siehe Fig. 5A und 5B).

Dabei kann eine mikrobielle Wirksamkeit sowohl durch das photokatalytische Prinzip wie am Beispiel von Titandoxid erreicht werden, als auch durch die antimikobielle Wirkung von Metallionen, welche durch Metallkolloide erzeugt und kontinuierlich an die Umgebung abgeben werden. Die Metallkolloide sind nur wenige Nanometer groß und durch ein besonderes Verhältnis von Größe und Oberfläche lässt sich ein Langzeiteffekt erzielen. Der Verbrauch der Metalle zu Metallionen ist sehr gering. Somit lässt sich eine mehrjährige Langzeitwirkung erzielen.

### Bezugszeichen

- 1: arterielle Blutleitung
- 2: Peristaltikpumpe
- 3: arterieller Druckabnehmer
- 4: Hochdruckblutleitung
- 5: arterieller Blutfänger
- 6: Zuleitung
- 7: Pumpe
- 8: Leitung
- 9: Dialysator
- 10: Dialysatzuleitung
- 11: Dialysatableitung
- 12: Blutableitung
- 13: venöser Druckfänger
- 14: Luftfalle
- 15: venöser Druckaufnehmer
- 16: venöse Blutleitung
- 17: Steuerungseinheit

## Patentansprüche

1. Medizintechnisches Gerät zur extrakorporalen Blutbehandlung, mit einer antimikrobiellen Oberflächenbeschichtung, **dadurch gekennzeichnet, dass** die antimikrobielle Oberflächenbeschichtung eine Pulverbeschichtung ist, die Additive mit antimikrobieller Wirkung aufweist, wobei die Additive metallorganische Stoffe sind, wobei die Additive eine ionisierende und photokatalytische Wirkung haben, sodass durch die Additive Ionen und Radikale gebildet werden, die jeweils von der Pulverbeschichtung emittiert werden.

2. Medizintechnisches Gerät nach Anspruch 1, **dadurch gekennzeichnet, dass** die Pulverbeschichtung auf eine metallische Oberfläche aufgebracht ist und das medizintechnische Gerät ein Gerätegehäuse aufweist und die Pulverbeschichtung zumindest teilweise auf dem Gerätegehäuse aufgebracht ist.

3. Medizintechnisches Gerät nach einem der vorstehenden Ansprüche, **dadurch gekennzeichnet, dass** in die Pulverbeschichtung die Additive eingebaut sind.

4. Medizintechnisches Gerät nach einem der vorstehenden Ansprüche, **dadurch gekennzeichnet, dass** die Additive mikrobiozid wirkende Oligomeren sind.

5. Medizintechnisches Gerät nach einem der vorstehenden Ansprüche, **dadurch gekennzeichnet, dass** die Pulverbeschichtung Trägerstoffe umfasst, an denen Ionenemittenten und/oder Katalysatoren anhaften.

6. Verfahren zur Bekämpfung von Mikroben und Mikroorganismen auf einer Oberfläche eines medizintechnischen Geräts nach einem der vorstehenden Ansprüche, wobei die Additive der Pulverbeschichtung des medizinischen Geräts Ionen und Radikale ausbilden und emittieren, die Stoffwechselsysteme von Mikroben und/oder Mikroorganismen angreifen.

7. Verfahren nach Anspruch 6, **dadurch gekennzeichnet, dass** die Additive fortwährend, vorzugsweise permanent, insbesondere kontinuierlich, Ionen und/oder Radikale bilden, die von der Pulverbeschichtung emittiert werden.

## Claims

1. A medical apparatus for extracorporeal blood treatment, comprising an antimicrobial surface coating, **characterized in that** the antimicrobial surface coating is a powder coating comprising additives having antimicrobial effect, wherein the additives are organometallic substances, wherein the additives have an ionizing and photocatalytic effect, so that ions and radicals are generated by the additives which are respectively emitted by the powder coating.

2. The medical apparatus according to claim 1, **characterized in that** the powder coating is applied onto a metallic surface and the medical apparatus comprises an apparatus case and the powder coating is at least partially applied onto the apparatus case.

3. The medical apparatus according to one of the preceding claims, **characterized in that** the additives are incorporated in the powder coating.

4. The medical apparatus according to one of the preceding claims, **characterized in that** the additives are oligomers with microbicide action.

5. The medical apparatus according to one of the preceding claims, **characterized in that** the powder coating comprises carrier substances to which ion emitters and/or catalyzers are adhered.

6. A method for controlling microbes and microorganisms on a surface of a medical apparatus according to one of the preceding claims, wherein the additives of the powder coating of the medical apparatus generate and emit ions and radicals which attack metabolic systems of microbes and/or microorganisms.

7. The method according to claim 6, **characterized in that** the additives generate ions and/or radicals, which are emitted by the powder coating, on a constant basis, preferably permanently, in particular in a continuous fashion.

## Revendications

1. Appareil technique médical de traitement du sang extracorporel avec un revêtement de surface antimicrobien, **caractérisé en ce que** le revêtement de surface antimicrobien est un revêtement en poudre qui présente des additifs avec une action antimicrobienne, dans lequel les additifs sont des substances organométalliques, dans lequel les additifs ont une action ionisante et photocatalytique si bien que sont formés par les additifs des ions et des radicaux qui sont émis respectivement par le revêtement en poudre.

2. Appareil technique médical selon la revendication 1, **caractérisé en ce que** le revêtement en poudre est appliqué sur une surface métallique et l'appareil technique médical présente un boîtier d'appareil et le revêtement en poudre est appliqué au moins en partie sur le boîtier d'appareil.

3. Appareil technique médical selon l'une quelconque des revendications précédentes, **caractérisé en ce que** les additifs sont intégrés dans le revêtement en poudre.

4. Appareil technique médical selon l'une quelconque des revendications précédentes, **caractérisé en ce que** les additifs sont des oligomères à action microbicide.

5. Appareil technique médical selon l'une quelconque des revendications précédentes, **caractérisé en ce que** le revêtement en poudre comprend des substances de support, auxquelles des émetteurs d'ions et/ou des catalyseurs adhèrent.

6. Procédé pour lutter contre des microbes et des micro-organismes sur une surface d'un appareil technique médical selon l'une quelconque des revendications précédentes, dans lequel les additifs du revêtement en poudre de l'appareil médical réalisent et émettent des ions et des radicaux qui attaquent des systèmes métaboliques de microbes et/ou de micro-organismes.

7. Procédé selon la revendication 6, **caractérisé en ce que** les additifs forment constamment, de préférence en permanence, en particulier en continu, des ions et/ou des radicaux qui sont émis par le revêtement en poudre.
